(19) Europäisches Patentamt
European Patent Office
Office européen des brevets

(11) Veröffentlichungsnummer: **0 026 464**
A2

(12) # EUROPÄISCHE PATENTANMELDUNG

(21) Anmeldenummer: 80105801.7

(22) Anmeldetag: 25.09.80

(51) Int. Cl.$^3$: **C 07 C 103/52**
A 61 K 37/02

(30) Priorität: 28.09.79 US 79630
28.04.80 US 144516

(43) Veröffentlichungstag der Anmeldung: 08.04.81 Patentblatt 81/14

(84) Benannte Vertragsstaaten: AT BE CH DE FR GB IT LI LU NL SE

(71) Anmelder: F. HOFFMANN-LA ROCHE & CO. Aktiengesellschaft

CH-4002 Basel(CH)

(72) Erfinder: Meienhofer, Johannes Arnold
35 Glenwood Road
Upper Montclair New Jersey 07043(US)

(74) Vertreter: Lederer, Franz, Dr. et al,
Patentanwälte Dr. Franz Lederer Reiner F. Meyer
Lucile-Grahn-Strasse 22
D-8000 München 80(DE)

(54) **Peptide, deren Herstellung, deren Verwendung und pharmazeutische Präparate.**

(57) Peptide der Formel

X-Glu-Asn-OH I

worin X H,
H-Ala-,
H-Glu-Ala-,
H-Glu-Glu-Ala-,
H-Val-Glu-Glu-Ala- oder
H-Val-Val-Glu-Glu-Ala- bedeutet,
und ihre physiologisch verträglichen Salze, deren Herstellung und Verwendung, sowie diese Verbindungen enthaltende pharmazeutische Präparate.

EP 0 026 464 A2

0026464

F.Hoffmann-La Roche & Co. Aktiengesellschaft,Basel,Schweiz

25. Sep. 1980

RAN 4105/58

# Peptide, deren Herstellung, deren Verwendung und pharmazeutische Präparate.

Die vorliegende Erfindung betrifft neue Peptide der allgemeinen Formel

X-Glu-Asn-OH      I

worin X H,
H-Ala-,
H-Glu-Ala-,
H-Glu-Glu-Ala-,
H-Val-Glu-Glu-Ala- oder
H-Val-Val-Glu-Glu-Ala- bedeutet,

und physiologisch verträgliche Salze davon, ein Verfahren zu ihrer Herstellung und diese enthaltende pharmazeutische Präparate.

Die obige allgemeine Formel I umfasst die folgenden Peptide:

H-Glu-Asn-OH,
H-Ala-Glu-Asn-OH,
H-Glu-Ala-Glu-Asn-OH,
H-Glu-Glu-Ala-Glu-Asn-OH,

Nt/17.9.80

H-Val-Glu-Glu-Ala-Glu-Asn-OH und
H-Val-Val-Glu-Glu-Ala-Glu-Asn-OH.

Besonders bevorzugte Verbindungen der obigen Formel I sind das Tripeptid und das Pentapeptid.

Die neuen erfindungsgemässen Verbindungen sind wertvolle pharmazeutische Wirkstoffe. Sie beeinflussen die Regulation, Differenzierung und Funktion von thymusabhängigen Lymphozyten.

Die erfindungsgemässen Peptide können durch herkömmliche Methoden, wie Flüssig- oder Festphasensynthese, leicht hergestellt werden.

Somit können Verbindungen der Formel I hergestellt werden, indem man aus einer Verbindung der Formel

$$Y\text{-}Glu(OR^1)\text{-}Asn\text{-}OR^2 \qquad II$$

worin Y A,
A-Ala-,
A-Glu($OR^1$)-Ala-,
A-Glu($OR^1$)-Glu($OR^1$)-Ala-,
A-Val-Glu($OR^1$)-Glu($OR^1$)-Ala oder
A-Val-Val-Glu($OR^1$)-Glu($OR^1$)-Ala;
A Wasserstoff oder eine herkömmliche α-Aminoschutzgruppe und
$R^1$ und $R^2$ herkömmliche Carboxyschutzgruppen bedeuten,

in an sich bekannter Weise die Schutzgruppen abspaltet und erwünschtenfalls den erhaltenen Stoff in ein physiologisch verträgliches Salz überführt.

Bevorzugte α-Aminoschutzgruppen sind die t-Butoxy-Gruppe (BOC) und die Benzyloxycarbonyl-Gruppe (Z).

Die Carboxygruppen in der Seitenkette von Glu und im C-Terminal Asn werden zweckmässigerweise als Ester

geschützt. Somit können $R^1$ und $R^2$ Aryl, vorzugsweise Phenyl (das durch niederes Alkyl, Halogen, Nitro, Mercapto oder niederes Alkylthio, wie Methylthio, substituiert sein kann); Aralkyl, vorzugsweise Benzyl (das durch Methoxy, Halogen oder Nitro substituiert sein kann); niederes Alkyl, wie Methyl, Ethyl, t-Butyl oder t-Amyl; oder substituiertes niederes Alkyl, wie 2-Haloethyl, 2-(Dimethylamino)-ethyl, Cyanomethyl, Benzhydryl oder Phenoxyl, bedeuten. Eine bevorzugte Carboxyschutzgruppe ist die t-Butylgruppe.

Die Abspaltung der Schutzgruppen aus einer Verbindung der Formel II kann in einem Schritt oder in zwei Schritten durchgeführt werden, beispielsweise durch Behandeln einer Verbindung der Formel II mit Fluorwasserstoff (HF), vorzugsweise in Gegenwart von Anisol oder durch katalytische Hydrierung.

Die katalytische Hydrierung wird vorzugsweise in Gegenwart von Palladium durchgeführt. Erwünschtenfalls kann man durch katalytische Hydrierung die α-Aminoschutzgruppe abspalten und anschliessend durch Behandeln mit einer Lösung von Trifluoressigsäure (TFA)/Methylenchlorid (1:1, v/v) die Carboxyschutzgruppen entfernen.

Die Herstellung der entsprechenden physiologisch verträglichen Salze kann nach allgemein üblichen und jedem Fachmann geläufigen Methoden erfolgen.

Die Ausgangsstoffe der Formel II können, sofern sie noch nicht bekannt sind (z.B. aus USP 4 148 788), nach bekannten und jedem Fachmann geläufigen Methoden hergestellt werden; und zwar in Analogie zu den in obiger US-Patentbeschreibung enthaltenen oder zu den weiter unten folgenden Beispielen.

Die Abspaltung der Schutzgruppen aus einer Verbindung der allgemeinen Formel II kann auch mittels Festphasen-Synthese erfolgen. Beispielsweise kann man eine an einen

Träger gebundene Verbindung der allgemeinen Formel

R-Ala-Glu(OBzl)-Asp(Co-NH-CH[$C_6H_5$]-$C_6H_4$-Träger)-OBzl    III

worin R Boc,
Boc-Glu(OBzl)-,
Boc-Glu(OBzl)-Glu(OBzl)-,
Boc-Val-Glu(OBzl)-Glu(OBzl)- oder
Boc-Val-Val-Glu(OBzl)-Glu(OBzl)- bedeutet,
mit wasserfreiem Fluorwasserstoff/Anisol behandeln. Durch diese Behandlung werden sämtliche Schutzgruppen in den Seitenketten entfernt, und das Peptid vom Träger freigesetzt. Die erhaltenen Lösungen, welche das rohe Peptid enthalten, werden anschliessend entsalzt; die Peptide werden nach bekannten und jedem Fachmann geläufigen Methoden gereinigt, beispielsweise durch Chromatographie. Es ist klar, das nicht nur die in der Formel III namentlich erwähnten Schutzgruppen, sondern alle in Festphasensynthesen für den Schutz von Glu und Asp geeigneten Schutzgruppen in obigem Verfahren verwendet werden können.

Die Ausgangsstoffe der obigen Formel III können hergestellt werden, indem man handelsübliches, an einen Träger gebundenes Benzhydrylamin neutralisiert und mit $N^{\alpha}$-Boc-α-benzyl-L-aspartat in Gegenwart von DCC acyliert. Das geschützte, an den Träger gebundene Asparagin wird anschliessend nach dem Verfahren von Wang [J.Amer. Chem.Soc.95, 1328 (1973)] benzoyliert, um die unsubstituierten Aminogruppen des Trägermaterials zu eliminieren, und in das Reaktionsgefäss eines automatischen Peptid-Syntheseapparats gegeben. Die Festphasensynthese wird anschliessend nacheinander mit den weiter unten aufgeführten geschützten Aminosäuren durchgeführt:

Boc-Glu(OBzl)-OH,
Boc-Ala-OH (Ende für Tripeptid)
Boc-Glu(OBzl)-OH (Ende für Tetrapeptid)
Boc-Glu(OBzl)-OH (Ende für Pentapeptid)

Boc-Val-OH (Ende für Hexapeptid)
Boc-Val-OH.

Für jeden Kupplungsschritt (120 Minuten) wird jeweils ein vierfacher Ueberschuss an Boc-geschützter Aminosäure und DCC verwendet. Die anschliessende Entfernung der Boc-Schutzgruppe erfolgt durch Behandeln mit 33-proz. Trifluoressigsäure (TFA) in Methylenchlorid während 30 Minuten.

Die erfindungsgemässen Verbindungen der Formel I beeinflussen die Regulierung, Differenzierung und Funktion von T-Zellen. Diese Wirkungen der erfindungsgemässen Verbindungen sind nun völlig überraschend, da aus dem Peptid-Hormon-Gebiet bekannt ist, dass biologisch aktive Peptide bereits durch Entfernen einer einzigen Aminosäure aus der Sequenz ihre biologische Wirksamkeit verlieren können. Dies gilt insbesondere für relativ kurze Peptid-Moleküle.

Die vorliegenden Verbindungen können warmblütigen Säugern parenteral, d.h. intravenös, subkutan oder intramuskulär, verabreicht werden. Bei intravenöser Verabreichung in einer täglichen Dosis von etwa 0,1 bis 50 mg/kg Körpergewicht sind diese Verbindungen starke immunpotenzierende Wirkstoffe. Natürlich hängen die obigen Dosierungen von den zu behandelnden Symptomen, ihrer Stärke und von der Behandlungsdauer ab. Eine zweckmässige pharmazeutische Dosiseinheit ist 1 bis 5 mg lyophilisierte Verbindung der Formel I, das kurz vor Gebrauch durch Zusatz von sterilem Wasser oder Kochsalzlösung in Lösung gebracht werden kann.

Die Erfindung umfasst auch die physiologisch verträglichen Salze von Verbindungen der Formel I und ihre Herstellung. Beispiele derartiger Salze sind das Natrium- oder Kaliumsalz, oder Salze mit starken organischen Basen, wie Guanidin. Ausserdem können die Präparate die Gegenionen zu den genannten Kationen enthalten, wie beispielsweise Chlorid, Bromid, Sulfat, Phosphat, Malat, Ascorbat oder

dergleichen.

Biologische Aktivität

A. Inkubation mit thymischen Präparationen oder Peptiden und Untersuchung der spezifischen Steroidbindung durch ganze Zellen.

Die AKR-hergeleitete, Ouabain-resistente T Lymphomalinie BW 5147 wurde in Dulbecco's modified Eagle medium (versetzt mit 50 Einheiten/ml Penicillin, 50 µg/ml Streptomycin, 2 mM Glutamin, 1 mM Natriumpyruvat und 10-proz. fetalem Kälberserum: Medium I) gezüchtet. In zweimonatigen Intervallen wurde die Linie in Gegenwart von $10^{-3}$ M Ouabain (Strophanthin G, Calbiochem) während 5 Tagen gezüchtet, um jede Ouabain-sensitive fremde Zelle zu eliminieren. Die im vorliegenden Experiment verwendeten Zellen wurden zu grosser Dichte gezüchtet (1,5-2 x $10^6$/ml).

Die Zellsuspensionen wurden zentrifugiert und dreimal bei 0-4°C mit HBSS gewaschen (alle Zentrifugationen wurden bei 180 x g während 5 Minuten durchgeführt). Die Inkubationen wurden mit verschiedenen Konzentrationen an Thymosin $\alpha_1$, Oktapeptid Glu-Val-Val-Glu-Glu-Ala-Glu-Asn, und den erfindungsgemässen Peptiden (Pentapeptid Glu-Glu-Ala-Glu-Asn und Tripeptid Ala-Glu-Asn) bei 37°C in einem befeuchteten $CO_2$-Inkubator (5% $CO_2$/95% Luft) durchgeführt, und zwar über verschieden lange Zeiträume, wie in den experimentellen Daten angegeben. Die Proben wurden in Medium I in Petrischalen vorbereitet (2,5-5 x $10^6$ Zellen/ml). Für die Messung der totalen ($^3$H) Dexamethasonbindung [($^3$H) dex] und der nicht-spezifischen ($^3$H) dex-Bindung durch ganze Zellen wurden dublikate Proben vorbereitet.

Für die Untersuchung der spezifischen Steroidbindung durch ganze Zellen wurde die Methode von Sibley und Tomkins, Cell 2, 221 (1974) modifiziert. Nach Inkubation mit Thymusfraktionen wurden die Dublikatkulturen in Polypropylenröhrchen geerntet. In Aliquoten wurde der Anteil lebender Zellen durch Trypanblau-Ausschluss in einem Hemacytometer ausgezählt. Die Zellen wurden zentrifugiert und bei 0-4°C dreimal mit jeweils 3 ml HBSS gewaschen. Die Zellen wurden in 1 ml Medium II suspendiert (RPMI 1640 versetzt mit 50 Einheiten/ml Penicillin, 50 µg/ml Streptomycin, 2 mM Glutamin, 10 mM HEPES, pH 7,4). Jede Duplikatkultur wurde jeweils mit $5 \times 10^{-9}$ M ($^3$H) dex versetzt. Eine dieser Kulturen erhielt zusätzlich einen 1000fachen Ueberschuss ($5 \times 10^{-6}$ M) an unmarkiertem dex (Sigma Chemical Co.) um die spezifische ($^3$H) dex-Bindung zu bestimmen. Diese wurde bestimmt durch Bildung der Differenz von gefundenem ($^3$H) dex zwischen Zellen, welche in Abwesenheit (total) und Zellen, welche in Anwesenheit (nicht-spezifisch) von unmarkiertem dex gezüchtet wurden. Nach Inkubation bei 37°C in einem befeuchteten $CO_2$-Inkubator (5% $CO_2$/95% Luft) während 45-50 Minuten wurden die Zellen zentrifugiert, bei 0-4°C zweimal mit je 3 ml HBSS gewaschen, bei 23°C in HBSS suspendiert und bei Raumtemperatur während 10 Minuten stehengelassen; anschliessend wurde zentrifugiert. Die Proben wurden in 0,2 ml Dulbecco's phosphate buffered saline (PBS) suspendiert und unter zweimaligem Nachwaschen mit je 0,2 ml PBS in ein Scintillations-Gefäss übergeführt. Nach Zugabe von Biofluor (New England Nuclear) wurden die Proben während jeweils 20 Minuten in einem Beckmann LS-250 Flüssig-Scintillationszähler ausgemessen. Die typische Standardabweichung der counts per min (CPM) betrug 0,7%.

Die in der Tabelle 1 zusammengefassten Resultate zeigen, dass das Tripeptid und das Pentapeptid der vorliegenden Erfindung die Umwandlung von precursor T-Zellen in Steroid-sensitive $T_1$-Zellen oder Steroid-resistente $T_2$-Zellen beeinflussen. Die Reaktivität ist vergleichbar mit derjenigen von Verbindungen und Präparationen, welche eine bekannte T-Zellen "maturation enhancing" Aktivität besitzen, wie beispielsweise Thymosin Fraktion 5, Thymosin $\alpha_1$ und das carboxylterminale Octapeptid von Thymosin $\alpha_1$ H-Glu-Val-Val-Glu-Glu-Ala-Glu-Asn-OH. Andererseits hat es sich gezeigt, dass Peptide von etwa der gleichen Kettenlänge wie die erfindungsgemässen Verbindungen, welche zum Teil

die selben Aminosäuren besitzen, völlig inaktiv sind, z.B. Leu-enkephalin (H-Tyr-Gly-Gly-Phe-Leu-OH) und H-Ala-Tyr-Met-Glu-OH.

Tabelle 1

| Peptid | Dosis [µg/ml] A | B | C | D | ($^3H$) Dex spezifisch gebunden [CPM/2 x $10^7$ Zellen] A | B | C | D |
|---|---|---|---|---|---|---|---|---|
| Keines | 0 | 0 | 0 | 0<br>0 | 6.901 | 10.352 | 9.865 | 10.567<br>10.646 |
| Thymosin $\alpha_1$ | 1.0 | | 0.5<br>5.0 | 0.1<br>0.5<br>5.0 | 7.703 | | 11.216<br>11.178 | 9.002<br>9.383<br>9.051 |
| Thymosin Fraktion 5 | | 100 | | | | 9.464 | | |
| H-Glu-Val-Val-Glu-Glu-Ala-Glu-Asn-OH | 0.1<br>0.5<br>1.0 | 0.5<br>1.0 | 0.5 | 0.01<br>0.1 | 7.467<br>7.858<br>7.835 | 9.103<br>9.804 | 11.614 | 9.093<br>9.772 |
| H-Ala-Glu-Asn-OH | | | 0.5<br>5.0 | 0.5 | | | 7.411<br>8.050 | 9.761 |
| H-Glu-Glu-Ala-Glu-Asn-OH | 0.1<br>0.5<br>1.0 | 0.5<br>1.0 | 0.5<br>5.0 | 0.5 | 7.109<br>7.850<br>7.422 | 9.370<br>9.055 | 9.219<br>7.866 | 9.812 |
| Leu-enkephalin | | 0.5<br>1.0 | | | | 10.005<br>10.056 | | |
| H-Ala-Tyr-Met-Glu-OH | | | 0.5<br>5.0 | 0.01<br>0.1<br>0.5 | | | 10.020<br>9.725 | 9.980<br>10.566<br>10.888 |

Inkubationszeiten: A = 44 Stunden; B = 41 Stunden; C = 42 Stunden; D = 16 Stunden

B. In zwei Experimenten (die Resultate sind in der Tabelle 2 zusammengefasst) wurden Mäuse-Thymus-Zellen, welche vorher mit Thymosinfraktion 5, Thymosin $\alpha_1$, H-Glu-Val-Val-Glu-Glu-Ala-Glu-Asn-OH, H-Glu-Val-Val-Glu-OH oder den erfindungsgemässen Peptiden behandelt wurden, mit Glucocorticoiden [Hydrocortison (HC) oder Dexamethason (Dex)] behandelt. Der Anteil der Zellen, welche den tötenden Effekt von Glucocorticoiden überlebt, ist bei den thymosin-behandelten Zellen höher als bei den unbehandelten Zellen. Optimale Konzentrationen für die Reaktion scheinen 100 µg Thymosinfraktion 5 und 1 µg Thymosin $\alpha_1$ pro ml Kulturmedium zu sein. Die erfindungsgemässen Peptide erhöhen ebenfalls den Anteil der überlebenden Zellen, währenddem H-Glu-Val-Val-Glu-OH, die N-terminale Hälfte des Octapeptides, keine Reaktion zeigt.

Tabelle 2: Effekt von Thymosin Fraktion 5, Thymosin $\alpha_1$ und Di- bis Oktapeptide auf Glucocorticoid-Resistenz von Mäuse-Thymus-Zellen unter serumfreien Bedingungen.

| Behandlung mit Verbindung | Konzentr. [µg/ml] | überlebende Zellen [%] Exp. 1 | Exp. 2 |
|---|---|---|---|
| Keine | 0 | 71.9 | 69.4 |
| Thymosin $\alpha_1$ | 0.1 | 85.2 | 82.7 |
| | 1.0 | 90.7 | 99.2 |
| Thymosin Fraktion 5 | 50 | 80.4 | |
| | 100 | 90.3 | |
| | 150 | 85.8 | |
| | 200 | 82.4 | |
| A | 0.1 | | 71.6 |
| | 0.5 | | 77.7 |
| B | 0.1 | | 66.5 |
| | 0.5 | | 77.1 |
| C | 0.1 | | 78.0 |
| | 0.5 | | 79.6 |
| D | 0.1 | | 74.1 |
| | 1.0 | | 85.5 |
| E | 0.1 | 98.3 | 84.3 |
| | 1.0 | 94.7 | 83.1 |
| F | 0.1 | 79.3 | 73.5 |
| | 1.0 | 84.7 | 80.1 |
| G | 0.1 | 87.9 | 85.9 |
| | 1.0 | 92.1 | 95.5 |
| H | 0.1 | | 73.7 |
| | 0.5 | | 73.9 |

A = H-Glu-Asn-OH

B = H-Ala-Glu-Asn-OH

C = H-Glu-Ala-Glu-Asn-OH

D = H-Glu-Glu-Ala-Glu-Asn-OH

E = H-Val-Glu-Glu-Ala-Glu-Asn-OH

F = H-Val-Val-Glu-Glu-Ala-Glu-Asn-OH

G = H-Glu-Val-Val-Glu-Glu-Ala-Glu-Asn-OH

H = H-Glu-Val-Val-Glu-OH

## Beispiel 1

### Herstellung von H-Ala-Glu-Asn-OH

220 mg H-Ala-Glu(OBzl)-Asn-OBzl• HCl werden in 15 ml Methanol, das 0,2 ml Essigsäure enthält, gelöst und in Gegenwart von 220 mg Palladium/Kohle (10%) während 3 Stunden bei Normaldruck und Raumtemperatur hydriert. Nach Abfiltrieren des Katalysators wird das Filtrat konzentriert und lyophilisiert. Ausbeute: 110 mg (74,7%) H-Ala-Glu-Asn-OH. Das Produkt ist bezüglich TLC [$R_f$ 0,47 (n-Propanol/Wasser, 7:3, v/v)] und HPLC einheitlich.

## Beispiel 2

### Herstellung von H-Ala-Glu-Asn-OH

31,93 g (0,143 Mol) N-Benzyloxycarbonyl-L-alanin werden in 280 ml Tetrahydrofuran gelöst und in einem 2-Liter 3-Hals-Rundkolben (versehen mit Thermometer und mechanischem Rührer) vorgelegt und in einem Trockeneis/Alkohol-Bad auf -20°C abgekühlt. Ueber einen Zeitraum von 2-3 Minuten werden unter Rühren 16,03 ml (0,143 Mol) N-Methylmorphin aus einem Tropftrichter zugegeben. Anschliessend versetzt man über einen Zeitraum von 2 Minuten bei -20°C mit 18,55 ml (0,143 Mol) Isobutylchlorformat. Man rührt während 4 Minuten bei -15°C und versetzt über einen Zeitraum von 10 Minuten portionenweise mit einer vorgekühlten Lösung von 44,6 g (0,119 Mol) H-Glu(OtBu)-Asn-OtBu in 280 ml THF, wobei die Temperatur unterhalb -15°C gehalten wird. Man rührt anschliessend während 30 Minuten bei -15°C und während 2 Stunden bei 25°C. Nach Eindampfen der Reaktionsmischung zur Trockne wird der Rückstand in 1,5 l Chloroform/0,75 l 10-proz. Natriumbicarbonatlösung aufgenommen [leichtes Erwärmen notwendig]. Die organische Phase wird nacheinander dreimal mit je 700 ml 10-proz. Natriumbicarbonatlösung und zweimal mit je 350 ml 10-proz. Kochsalzlösung gewaschen. Die wässrigen Phasen werden dreimal mit je 300 ml Chloroform

zurückextrahiert. Die vereinigten organischen Phasen werden über Magnesiumsulfat getrocknet, filtriert und im Vakuum eingedampft. Man erhält 85,2 g rohes N-Benzyloxycarbonyl-L-alanyl-γ-t-butyl-L-glutamyl-L-asparagin-t-butylester, das aus Essigester kristallisiert. Ausbeute: 56,40 g (81,7%); Smp. 164,5-167°C; $R_f$ 0,28 (Chloroform/Methanol/Ethanol, 80:20:5, v/v); $[\alpha]_D^{25}$ -34,44° (c 1, Methanol).

56,3 g (0,0974 Mol) N-Benzyloxycarbonyl-L-alanyl-γ-t-butyl-L-glutamyl-L-asparagin-t-butylester werden in 600 ml Methanol gelöst und in Gegenwart von 11 g Palladium/Bariumsulfat (5%) in einem Vibromixer während 3 Stunden hydriert. Die Reaktionsmischung wird über Kieselgel filtriert, mit 100 ml Methanol gewaschen und zu einem braunen Oel eingedampft. Der Rückstand kristallisiert aus Essigester/Petrolether und liefert 41,93 g (96,8%) weisses kristallines L-Alanyl-γ-t-butyl-L-glutamyl-L-asparagin-t-butylester in 3 Portionen; Smp. 121-123°C; $R_f$ 0,18 (Chloroform/Methanol/Essigsäure, 80:20:5, v/v); $[\alpha]_D^{25}$ - 20,32° (c 1, Methanol).

500 mg (1,13 mMol) L-Alanyl-γ-t-butyl-L-glutamyl-L-asparagin-t-butylester werden in einem 50 ml Rundkolben vorgelegt und mit einer Lösung von 5 ml TFA in 5 ml Methylenchlorid behandelt. Man rührt während 3 Stunden bei 25°C unter Stickstoff. Die Reaktionsmischung wird im Vakuum eingedampft. Der Rückstand wird mit Ether behandelt, getrocknet und in 2-proz. Ammoniumacetatlösung (pH 8,1) aufgenommen und auf eine mit 2-proz. Ammoniumacetatlösung (pH 8,1) equilibrierte Bio-Rad AG 1-x2®-Kolonne (1,5 x 20 cm, $OH^-$-Form) aufgetragen. Man eluiert mit einem Gradienten [0,02 M bis 1,0 M Essigsäure] und sammelt Fraktionen zu 100 Tropfen (5,7 ml). Die Produkt enthaltenden Fraktionen werden durch TLC von Aliquoten der einzelnen Fraktionen bestimmt. Die Fraktionen 9-16 (51-91 ml) werden vereinigt und lyophilisiert. Man erhält 226 mg (60,4%) weisses H-Ala-Glu-Asn-OH vom Smp. 178°C (Zers.); $R_f$ 0,47 (n-Butanol/Essigsäure/ Essigester/Wasser; 1:1:1:1, v/v); $[\alpha]_D^{25}$ -20,47° (c 1,1, 0,1 M Salzsäure).

## Beispiel 3

### Herstellung von H-Glu-Glu-Ala-Glu-Asn-OH

100 mg des geschützten Pentapeptids H-Glu(OBzl)-Glu(OBzl)-Ala-Glu(OBzl)-Asn-OBzl werden in 6 ml Methanol, das 2 Tropfen Essigsäure enthält, gelöst und in Gegenwart von 200 mg Palladium/Kohle (10%) während 2,5 Stunden bei Normaldruck und Raumtemperatur hydriert. Der Katalysator wird abfiltriert; das Filtrat wird konzentriert und lyophilisiert. Ausbeute: 48 mg (80%) H-Glu-Glu-Ala-Glu-Asn-OH. Die Reinigung erfolgt durch Anionenaustauscherchromatographie mittels einer Bio-Rad AG 1-X2®-Kolonne. Das Produkt ist bezüglich TLC [$R_f$ 0,19 (n-Propanol/Wasser, 7:3, v/v)] und HPLC einheitlich.

## Beispiel 4

### Herstellung von H-Glu-Asn-OH

Eine Lösung von 50,7 (0,15 Mol) N-Benzyloxycarbonyl-γ-t-butyl-L-glutamat in 300 ml frisch destilliertem Tetrahydrofuran (THF) wird in einem mit Thermometer, mechanischem Rührer und Tropftrichter versehenen 2-Liter 3-Hals-Rundkolben vorgelegt, in einem Trockeneis/Alkohol-Bad auf -15°C abgekühlt und tropfenweise mit 16,8 ml (0,15 Mol) N-Methylmorpholin versetzt. Ueber einen Zeitraum von 2 Minuten versetzt man anschliessend bei -15°C mit 19,7 ml (0,15 Mol) Isobutylchloroformat. Man rührt noch 2 Minuten bei -15°C und gibt anschliessend über einen Zeitraum von 4 Minuten tropfenweise eine vorgekühlte Lösung von 25,6 g (0,136 Mol) L-Asparagin-t-butylester in 270 ml THF und 30 ml Dimethylformamid (DMF) hinzu, wobei die Temperatur bei -15°C gehalten wird. Man rührt die Reaktionsmischung während 30 Minuten bei -15°C und während 2,5 Stunden bei 25°C und dampft anschliessend im Vakuum ein. Der Rückstand wird in Essigester gelöst und nacheinander zweimal mit je 300 ml 10-proz. Natriumbicarbonatlösung, 30 ml gesättigter

Kochsalzlösung, zweimal mit je 300 ml 1 M Zitronensäurelösung und 300 ml gesättigter Kochsalzlösung gewaschen. Die organische Phase wird über Magnesiumsulfat getrocknet, filtriert und im Vakuum eingedampft, wobei ein dickflüssiges Oel anfällt. Durch Kristallisieren aus Essigester/Ether erhält man 34,5 g N-Benzyloxycarbonyl-γ-t-butyl-L-glutamyl-L-asparagin-t-butylester. Durch Behandeln der Mutterlauge mit Ether erhält man weitere 31,1 g Produkt. Gesamtausbeute: 65,6 g (95%); Smp. 139°C (Zers.); $R_f$ 0,22 (Chloroform/Methanol/Essigsäure; 80:2:0,4, v/v). Durch Umkristallisieren aus Methanol/Ether erhält man eine analysenreine Probe von Smp. 142-144,5°C; $[\alpha]_D^{25}$ -19,14° (c 1, Methanol).

65,6 g (0,13 Mol) N-Benzyloxycarbonyl-γ-t-butyl-L-glutamyl-L-asparagin-t-butylester werden in 580 ml Methanol aufgelöst und in Gegenwart von 15 g Palladium/Bariumsulfat (5%) während 3 Stunden in einem Vibromixer hydriert. Die Reaktionsmischung wird über Kieselgur filtriert und im Vakuum eingedampft wobei man 45,2 g (93%) γ-tert-Butyl-L-glutamyl-L-asparagin-t-butylester erhält; $R_f$ 0,22 (Chloroform/Methanol/Essigsäure, 85:10:5, v/v). Durch Umkristallisieren aus Essigester/Petrolether erhält man eine analysenreine Probe von Smp. 118-120°C; $[\alpha]_D^{25}$ -4,46° (c 1, Methanol).

0,50 g (1,34 mMol) γ-t-Butyl-L-glutamyl-L-asparagin-t-butylester werden in einem 50 ml Rundkolben vorgelegt, mit einer Lösung von 5 ml TFA in 5 ml Methylenchlorid versetzt und während 2 Stunden bei 25°C unter Stickstoff gerührt. Nach Eindampfen der Reaktionsmischung im Vakuum wird der Rückstand mit Ether behandelt und im Vakuum getrocknet. Dieses Material wird in 2-proz. Ammoniumacetatlösung (pH 8,1) gelöst und auf eine mit 2-proz. Ammoniumacetatlösung (pH 8,1) äquilibrierte Bio-Rad AG 1-X2® Kolonne (1,5 x 20 cm, $OH^-$-Form) gebracht. Man eluiert mit einem Gradienten [0,02M bis 1,0M Essigsäure] und sammelt Fraktionen zu 100 Tropfen (5,7 ml). Die Produkt enthaltenden Fraktionen

werden durch TLC von Aliquoten der einzelnen Fraktionen bestimmt. Die Fraktionen 11-23 (63-122 ml) werden vereinigt und lyophilisiert. Man erhält 254 mg (67,9%) weisses H-Glu-Asn-OH vom Smp. 189,5-190,6°C; $R_f$ 0,53 (n-Butanol/Essigsäure/Essigester/Wasser, 1:1:1:1, v/v); $[\alpha]_D^{25}$ + 7,25° (c 1, 0,01M Salzsäure).

Beispiel 5

Herstellung von H-Glu-Ala-Glu-Asn-OH

58,1 g (0,112 Mol N-Benzyloxycarbonyl-γ-t-butyl-L-glutamat DCHA (DCHA = Dicyclohexylamin) werden in einer Mischung aus 375 ml Essigester und 375 ml 0,5M Schwefelsäure gelöst. Die organische Phase wird abgetrennt und zweimal mit je 150 ml 0,5N Schwefelsäure extrahiert. Die vereinigten wässrigen Auszüge werden zweimal mit je 150 ml Essigester zurückextrahiert. Die organischen Auszüge werden über Magnesiumsulfat getrocknet, filtriert und im Vakuum zu einem gelben Oel eingedampft. Der Rückstand, Z-Glu(OtBu)-OH, wird in 282 ml THF aufgenommen und in einem 2-Liter 3-Hals-Rundkolben (mit Thermometer und mechanischem Rührer versehen) vorgelegt und in einem Trockeneis/Alkoholbad auf -20°C abgekühlt. Man versetzt tropfenweise mit 12,55 ml (0,112 Mol) N-Methylmorpholin. Anschliessend versetzt man tropfenweise über einen Zeitraum von 2 Minuten mit 14,53 ml (0,112 Mol) Isobutylchloroformat wobei man die Temperatur auf -20°C hält. Man rührt noch während 4 Minuten bei -20°C und versetzt über einen Zeitraum von 5 Minuten portionenweise mit einer vorgekühlten Lösung von 41,42 g (0,0933 Mol) L-Alanyl-γ-t-butyl-L-glutamyl-L-asparagin-t-butylester in 293 ml THF und 240 ml DMF wobei man die Temperatur bei -20°C hält. Man rührt noch während 30 Minuten bei -15°C und anschliessend während 16 Stunden bei 25°C.

Nach Eindampfen der Reaktionsmischung im Vakuum wird der Rückstand zwischen 1,2 Liter Chloroform und 0,6 Liter 10-proz. Natriumbicarbonatlösung verteilt [leichtes Erwärmen notwendig]. Die organische Phase wird dreimal mit je 550 ml 10-proz. Natriumbicarbonatlösung und zweimal mit je 280 ml gesättigter Kochsalzlösung extrahiert. Die vereinigten wässrigen Phasen werden dreimal mit je 240 ml Chloroform zurückextrahiert. Die vereinigten organischen Auszüge werden über Magnesiumsulfat getrocknet, filtriert und im Vakuum eingedampft. Man erhält H-Glu-Ala-Glu-Asn-OH als weissen Festkörper der aus Essigester/ DMF/Petrolether (475 ml: 200 ml: 200 ml) kristallisiert. Man erhält 63,03 g (88,6%) weisses kristallines Produkt vom Smp. 211-212,5°C; $R_f$ 0,89 (Chloroform/Methanol/Essigsäure, 80:10:5, v/v); $[\alpha]_D^{25}$ -15,36° (c 1, DMF).

63,03 g (0,0814 Mol) Z-Glu(OtBu)-Ala-Glu(OtBu)-Asn-OtBu werden in 559 ml DMF gelöst und in Gegenwart von 14,4 g Palladium/Bariumsulfat (5%) während 3 Stunden in einem Vibromixer hydriert. Die Reaktionsmischung wird über Kieselgur filtriert, mit 100 ml DMF/Methanol gewaschen und im Vakuum zu einem Oel eingedampft. Der Rückstand kristallisiert aus Ether/Petrolether, wobei 48,46 g (94,4%) H-Glu(OtBu)-Ala-Glu(OtBu)-Asn-OtBu als weisser Festkörper in 2 Portionen anfallen; Smp. 161-164°C; $R_f$ 0,70 (n-Butanol/Essigsäure/Essigester/Wasser, 1:1:1:1, v/v); $R_f$ 0,26 (Chloroform/Methanol/Essigsäure, 80:20:5, v/v); $[\alpha]_D^{25}$ -29,55° (c 1, Methanol).

500 mg (0,794 mMol) H-Glu(OtBu)-Ala-Glu(OtBu)-Asn-OtBu werden in einem 50 ml Rundkolben vorgelegt, mit einer Lösung von 5 ml TFA in 5 ml Methylenchlorid versetzt und während 3 Stunden bei 25°C unter Stickstoff gerührt. Nach Eindampfen der Reaktionsmischung im Vakuum wird der Rückstand mit Ether behandelt und getrocknet. Man erhält 548 mg Rohprodukt. Davon werden 297 mg in 2-proz. Ammoniumacetatlösung (pH 8,1) aufgenommen und auf eine mit 2-proz. Ammoniumacetatlösung (pH 8,1) equilibrierte Bio-Rad AG 1-X2®-Kolonne (1,5 x 20 cm, $OH^-$-Form) gegeben. Man eluiert mit einem Gradienten [0,02M bis 1,0M Essigsäure] und sammelt Fraktionen zu 100 Tropfen (5,7 ml). Die Produkt enthaltenden Fraktionen

werden durch TLC von Aliquoten der einzelnen Fraktionen bestimmt. Die Fraktionen 24-38 (137-217 ml) werden vereinigt und lyophilisiert. Man erhält 159 mg (80,3%) H-Glu-Ala-Glu-Asn-OH als weissen Festkörper vom Smp. 211,5-214,5° (Zers.); $R_f$ 0,47 (n-Butanol/Essgisäure/Essigester/Wasser, 1:1:1:1, v/v); $[\alpha]_D^{25}$ -37,25° (c 1, 0,1M Salzsäure).

Beispiel 6

Herstellung von H-Val-Glu-Glu-Ala-Glu-Asn-OH

52,4 g (0,21 Mol) N-Benzyloxycarbonyl-L-valin werden in 400 ml frisch destillierten THF gelöst und in einem 2-Liter 3-Hals-Rundkolben (versehen mit Thermometer und Tropftrichter) vorgelegt und in ein Trockeneis/Alkohol-Bad auf -15°C abgekühlt. Bei dieser Temperatur versetzt man tropfenweise unter Rühren mit 23 ml (0,21 Mol) N-Methylmorpholin. Anschliessend gibt man über einen Zeitraum von 2 Minuten tropfenweise 27,6 ml (0,21 Mol) Isobutylchlorformat hinzu, wobei die Temperatur bei -15°C gehalten wird. Man rührt 2 Minuten bei -15°C und versetzt anschliessend tropfenweise mit einer vorgekühlten Lösung von 60,4 g (0,21 Mol) L-Glutaminsäure-α-methylester-γ-t-butylester in 300 ml THF und 80 ml DMF unter gleichzeitiger Zugabe von 23,5 ml (0,21 Mol) N-Methylmorpholin. Die Additionszeit beträgt 5 Minuten, wobei man die Temperatur bei -15°C hält. Der Reaktionsmischung wird anschliessend während 30 Minuten bei -15°C und dann während 2,5 Stunden bei 25°C gerührt und im Vakuum eingedampft. Der Rückstand wird in 600 ml Essigester aufgenommen und dreimal mit je 100 ml 10-proz. Natriumbicarbonatlösung, dreimal mit je 100 ml 1M Zitronensäure und mit 100 ml gesättigter Kochsalzlösung gewaschen. Die wässrigen Phasen werden mit Essigester zurückextrahiert. Die vereinigten organischen Auszüge werden über Magnesiumsulfat getrocknet, filtriert und auf 100 ml eingeengt. Man versetzt mit Petrolether. Der ausgefallene Festkörper wird abfiltriert und im Vakuum getrocknet. Man erhält 73,9 g (78%) Z-Val-Glu(OtBu)-OMe. Durch Umkristallisieren aus

Tetrachlorkohlenstoff/Petrolether erhält man 66,7 g weisses kristallines Produkt vom Smp. 66,5-68°C; $R_f$ 0,74 (n-Butanol/Essigsäure/Essigester/Wasser, 1:1:1:1, v/v); $[\alpha]_D^{25}$ -28,91° (c 1, Methanol).

0,50 g Z-Val-Glu(OtBu)-OMe werden in 10 ml DMF und 10 ml n-Butanol gelöst und in einem 50 ml Rundkolben vorgelegt. Anschliessend versetzt man mit 5,3 ml Hydrazin-Hydrat und rührt die Mischung während 17 Stunden bei 25°C. Nach Eindampfen der Reaktionsmischung im Vakuum wird der Rückstand aus Isopropanol kristallisiert, wobei mehrere Portionen anfallen. Man erhält 0,256 g (51%) Z-Glu(OtBu)-Ala-Glu(OtBu)-Asn-OtBu vom Smp. 143-145°C; $R_f$ 0,83 (n-Butanol/Essigsäure/Essigester/Wasser, 1:1:1:1, v/v); $R_f$ 0,64 (Chloroform/Methanol/Essigsäure, 80:5:1, v/v); $[\alpha]_D^{25}$ -33,0° (c 1, Methanol).

63,03 g (0,0814 Mol) Z-Glu(OtBu)-Ala-Glu(OtBu)-Asn-OtBu werden in 559 ml DMF gelöst und in Gegenwart von 14,4 g Palladium/Bariumsulfat (5%) während 3 Stunden in einem Vibromixer hydriert. Die Reaktionsmischung wird über Kieselgur filtriert, mit 100 ml DMF/Methanol gewaschen und im Vakuum zu einem Oel eingedampft. Durch Kristallisieren des Rückstandes aus Ether/Petrolether erhält man 48,46 g (94,4%) H-Glu(OtBu)-Ala-Glu(OtBu)-Asn-OtBu vom Smp. 161-164°C; $R_f$ 0,70 (n-Butanol/Essigsäure/Essigester/Wasser, 1:1:1:1, v/v); $R_f$ 0,26 (Chloroform/Methanol/Essigsäure, 80:20:5, v/v); $[\alpha]_D^{25}$ -29,55° (c 1, Methanol).

0,441 g (0,98 mMol) Z-Val-Glu(OtBu)-$NHNH_2$ werden in 12 ml DMF gelöst und in einem 50 ml 3-Hals-Rundkolben (versehen mit Thermometer, Tropftrichter und Trockenrohr) vorgelegt und in einem Trockeneis/Alkohol-Bad auf -20°C abgekühlt. Die Lösung wird mit 1,9 ml 3,1N Salzsäure in Tetrahydrofuran und 0,196 ml Isoamylnitrit behandelt und während 30 Minuten bei -20°C gerührt. Nach Abkühlen auf -25°C wird mit 0,82 ml Triethylamin versetzt. Nach Einstellen der Temperatur auf -20°C versetzt man mit einer Lösung von H-Glu(OtBu)-Ala-Glu(OtBu)-Asn-OtBu in 6 ml Dimethylformamid und anschliessend mit 0,16 ml Triethylamin. Der pH der Lösung wird durch periodische Zugabe von Triethylamin bei 8,0 gehalten. Die Reaktionsmischung wird während 30 Minuten bei -20°C und anschliessend 16 Stunden bei 7°C und dann 5 Stunden bei 25°C gerührt. Nach Eindampfen im Vakuum wird der Rückstand mit warmem Tetrahydrofuran behandelt. Den zurückbleibenden Festkörper wäscht man viermal mit je 30 ml Wasser und vereinigt den Rückstand mit dem Produkt, das man aus der THF-Waschlösung erhalten hat. Durch Umkristallisieren aus Methanol erhält man 0,469 g (45,5%) Z-Val-Glu(OtBu)-Glu(OtBu)-Ala-Glu(OtBu)-Asn-OtBu vom Smp. 217-219°C; $R_f$ 0,45 (Chloroform/Methanol/Essigsäure, 80:5:1, v/v); $[\alpha]_D^{25}$ -16,19° (c 1, Dimethylsulfoxid).

0,418 g (0,4 mMol) Z-Val-Glu(OtBu)-Glu(OtBu)-Ala-Glu (OtBu)-Asn-OtBu werden in 30 ml DMF gelöst und in Gegenwart von 0,4 g Palladium/Bariumsulfat (5%) während 3 Stunden in einem Vibromixer hydriert. Die Reaktionsmischung wird über Kieselgur filtriert, mit 30 ml DMF gewaschen und im Vakuum eingedampft. Man erhält einen weissen Schaum, der nach Umkristallisieren aus Isopropanol/Petrolether 0,351 g (96%) H-Val-Glu(OtBu)-Glu(OtBu)-Ala-Glu(OtBu)-Asn-OtBu als weissen Festkörper liefert; Smp. 227° (Zers.) ; $R_f$ 0,71 (n-Butanol/ Essigester/Wasser/Essigsäure, 1:1:1:1, v/v); $[\alpha]_D^{25}$ -18,86° (c 1, Dimethylsulfoxid).

0,310 g (0,33 mMol) H-Val-Glu(OtBu)-Glu(OtBu)-Ala-Glu(OtBu)-Asn-OtBu werden in einem 15 ml Rundkolben vorgelegt, mit einer Lösung von 2,5 ml TFA in 2,5 ml Methylenchlorid versetzt und während 3 Stunden bei 25°C unter Stickstoff gerührt. Die Reaktionsmischung wird im Vakuum eingedampft; der Rückstand wird in möglichst wenig 2-proz. Ammoniumacetatlösung (pH 8,1) gelöst und auf eine Dowex 1-X2®-Kolonne (1,5 x 20 cm) gegeben.[Kolonne ($OH^-$-Form) vorher mit 2-proz. Ammoniumacetatlösung (pH 8,1) equilibriert]. Man eluiert mit einem Gradienten (0,01M bis 0,1M Essigsäure) und sammelt Fraktionen zu 100 Tropfen (5,7 ml). Die Produkt

enthaltenden Fraktionen werden durch TLC von Aliquoten der einzelnen Fraktionen bestimmt (Fluorescamin spray procedure). Die Fraktionen 56-67 (319-382 ml) werden vereinigt und lyophilisiert. Man erhält 0,134 g (59%) H-Val-Glu-Glu-Ala-Glu-Asn-OH als weissen Festkörper vom Smp. 244-245°C (Zers.); $R_f$ 0,50 (n-Butanol/Essigester/Wasser/Essigsäure, 1:1:1:1, v/v); $R_f$ 0,29 (n-Butanol/Essigsäure/Pyridin/Wasser, 15:3:10:12, v/v); $[\alpha]_D^{25}$ -67,77° (c 0,8, 0,1M Salzsäure).

## Beispiel 7

### Herstellung von H-Val-Val-Glu-Glu-Ala-Glu-Asn-OH

33,9 g (75,3 mMol) N-Benzyloxycarbonyl-L-valyl-γ-t-butyl-L-glutaminsäure-methylester werden in 40 ml DMF und 150 ml THF gelöst und in Gegenwart von 7,5 g Palladium/Bariumsulfat (5%) während 2,5 Stunden in einem Vibromixer hydriert. Die Reaktionsmischung wird über Kieselgur filtriert und auf 0°C abgekühlt. Die erhaltene Lösung von L-Valyl-γ-t-butyl-L-glutaminsäure-methylester wird zur Weiterverwendung in einem Gefrierschrank aufbewahrt.

22,7 g (90,4 mMol) N-Benzyloxycarbonyl-L-valin wird in 20 ml frisch destilliertem THF gelöst und in einem 1-Liter 3-Hals-Rundkolben (mit Thermometer, mechanischem Rührer und Tropftrichter versehen) vorgelegt und in einem Trockeneis/Alkohol-Bad auf -15°C abgekühlt. Bei dieser Temperatur versetzt man unter Rühren mit 10,1 ml (90,4 mMol) N-Methylmorpholin. Anschliessend versetzt man über einen Zeitraum von 2 Minuten, tropfenweise mit 11,9 ml (90,4 mMol) Isobutylchloroformat. Man rührt weitere 2 Minuten bei -15°C und versetzt über einen Zeitraum von 4,5 Minuten mit einer vorgekühlten Lösung (-20°C) von 75,3 mMol L-Valyl-γ-t-butyl-L-glutaminsäure-methylester (siehe oben). Die Reaktionsmischung wird während 30 Minuten bei -15°C und anschliessend während 17 Stunden bei 25°C gerührt und im Vakuum eingedampft. Der Rückstand wird im Chloroform aufgenommen und viermal mit je 250 ml 10-proz. Natriumbicarbonatlösung,

200 ml gesättigter Natriumchloridlösung, zweimal mit je 200 ml 1M Zitronensäure und mit 200 ml gesättigter Kochsalzlösung gewaschen. Die wässrigen Phasen werden mit Chloroform zurückextrahiert. Die vereinigten organischen Auszüge werden über Magnesiumsulfat getrocknet, filtriert und im Vakuum eingedampft. Durch Umkristallisieren des erhaltenen Produktes aus Propanol erhält man 25,4 g (62%) Z-Val-Val-Glu(OtBu)-OMe als weissen kristallinen Festkörper vom Smp. 203-204°C; $R_f$ 0,47 (Chloroform/Methanol/Essigsäure, 80:2:0,4, v/v); $[\alpha]_D^{25}$ -7,40° (c 1, DMF).

0,50 g (0,91 mMol) Z-Val-Val-Glu(OtBu)-OMe werden in 10 ml DMF und 10 ml n-Butanol gelöst und unter Rühren bei 25°C mit 4,3 ml (0,09 Mol) Hydrazinhydrat versetzt. Die Reaktionsmischung wird während 17 Stunden gerührt und im Vakuum eingedampft. Der Rückstand wird in ca. 10 ml warmem Methanol aufgenommen. Man versetzt mit Wasser, filtriert das ausgefallene Material ab und trocknet. Man erhält 0,456 g (91%) Z-Val-Val-Glu(OtBu)-$NHNH_2$ als weissen Festkörper vom Smp. 221°C (Zers.); $R_f$ 0,38 (Chloroform/Methanol/Essigsäure, 80:5:1, v/v); $[\alpha]_D^{25}$ -0,56° (c 1, Dimethylsulfoxid).

Eine Suspension von 0,275 g (0,5 mMol) Z-Val-Val-Glu(OtBu)-$NHNH_2$ in 6 ml Dimethylformamid wird in einem 50 ml 3-Hals-Rundkolben (mit Thermometer und Trockenrohr versehen) vorgelegt und in einem Trockeneis/Alkohol-Bad auf -20°C abgekühlt. Die Mischung wird mit 1,0 ml (3 mMol) 2,9N Salzsäure/Tetrahydrofuran behandelt. Nach Zugabe von 0,10 ml (0,72 mMol) Isoamylnitrit rührt man noch während 30 Minuten bei -20°C. Die Reaktionsmischung wird anschliessend auf -25°C abgekühlt und mit 0,42 ml (3 mMol) Triethylamin versetzt. Nach Einstellen der Temperatur auf -20°C versetzt man zuerst mit einer Lösung von 0,346 mg (0,55 mMol) H-Glu(OtBu)-Ala-Glu(OtBu)-Asn-OtBu in 3 ml Dimethylformamid und dann mit 0,08 ml (0,55 mMol) Triethylamin. Durch periodische Zugabe von Triethylamin wird der pH der Lösung bei 8,0 gehalten. Anschliessend rührt man noch 30 Minuten bei -20°C, während 16 Stunden bei 7°C und während 5 Stunden bei 25°C.

Die Reaktionsmischung wird im Vakuum eingedampft; der Rückstand wird mit Wasser behandelt. Der verbleibende Festkörper wird im Vakuum getrocknet; man erhält 0,495 g (85%) Z-Val-Val-Glu(OtBu)-Glu(OtBu)-Ala-Glu(OtBu)-Asn-OtBu. Durch Umkristallisieren des Produktes aus Methanol/Wasser erhält man 0,423 g (73,7%) Produkt vom Smp. 230,5-231,5°C (Zers.); $R_f$ 0,88 (n-Butanol/Essigester/Wasser/Essigsäure, 1:1:1:1, v/v); $[\alpha]_D^{25}$ -19,20° (c 1, Dimethylsulfoxid).

0,308 g (0,27 mMol) Z-Val-Val-Glu(OtBu)-Glu(OtBu)-Ala-Glu(OtBu)-Asn-OtBu werden in 35 ml Dimethylformamid gelöst und in Gegenwart von 0,36 g Palladium/Kohle (10%) während 2 Stunden in einem Vibromixer hydriert. Die Reaktionsmischung wird über Kieselgur filtriert und im Vakuum eingedampft. Der Rückstand wird in Isopropanol aufgenommen. Man versetzt mit Wasser und filtriert den ausgefallenen weissen Festkörper ab. Man erhält 0,231 g (84,3%) H-Val-Val-Glu(OtBu)-Glu(OtBu)-Ala-Glu(OtBu)-Asn-OtBu vom Smp. 229-231°C; $R_f$ 0,74 (n-Butanol/Essigester/Wasser/Essigsäure, 1:1:1:1, v/v); $R_f$ 0,53 (n-Butanol/Essigsäure/Wasser, 4:1:1, v/v).

146 mg (0,144 mMol) H-Val-Val-Glu(OtBu)-Glu(OtBu)-Ala-Glu(OtBu)-Asn-OtBu werden in einem 10 ml Rundkolben vorgelegt und mit einer Mischung aus 2,5 ml TFA und 2,5 ml Methylenchlorid versetzt. Man rührt während 3 Stunden bei 25°C unter Stickstoff. Die Reaktionsmischung wird im Vakuum eingedampft; der Rückstand wird in 2-proz. Ammoniumacetatlösung (pH 8,1) aufgenommen und auf eine mit 2-proz. Ammoniumacetatlösung equilibrierte Bio-Rad AG 1-X2®-Kolonne (1,5 x 20 cm, $OH^-$-Form) gegeben. Man eluiert mit einem Gradienten [0,02M bis 1,0M Essigsäure] und sammelt Fraktionen zu 50 Tropfen (2,8 ml). Die Produkt enthaltenden Fraktionen werden durch TLC von Aliquoten der einzelnen Fraktionen bestimmt. Die Fraktionen 88-99 (246-277 ml) werden vereinigt und lyophilisiert. Man erhält 48 mg (40,3%) H-Val-Val-Glu-Glu-Ala-Glu-Asn-OH als weissen Festkörper vom Smp. 235-238°C (Zers.); $R_f$ 0,58 (n-Butanol/Essigsäure/Essigester/Wasser, 1:1:1:1, v/v); $R_f$ 0,22

(n-Butanol/Essigsäure/Pyridin/Wasser, 15:3:10:12, v/v); $[\alpha]_D^{25}$ -75,59° (c 0,8, 0,1M Salzsäure).

## Patentansprüche

1. Peptide der Formel

$$X\text{-}Glu\text{-}Asn\text{-}OH \qquad I$$

worin X H,
H-Ala-,
H-Glu-Ala-,
H-Glu-Glu-Ala-,
H-Val-Glu-Glu-Ala- oder
H-Val-Val-Glu-Glu-Ala-
bedeutet und physiologisch verträgliche Salze davon.

2. H-Glu-Asn-OH.

3. H-Ala-Glu-Asn-OH.

4. H-Glu-Ala-Glu-Asn-OH.

5. H-Glu-Glu-Ala-Glu-Asn-OH.

6. H-Val-Glu-Glu-Ala-Glu-Asn-OH.

7. H-Val-Val-Glu-Glu-Ala-Glu-Asn-OH.

8. Peptide gemäss Anspruch 1 oder physiologisch verträgliche Salze davon als pharmazeutische Wirkstoffe.

9. Peptide gemäss Anspruch 1 oder physiologisch verträgliche Salze davon als die Regulierung, Differenzierung und Funktion thymusabhängiger Lymphozyten beeinflussende Wirkstoffe.

10. Verfahren zur Herstellung der in Anspruch 1 definierten Verbindungen der Formel I und ihrer physiologisch verträglichen Salze, dadurch gekennzeichnet, dass man aus einer Verbindung der Formel

$$Y\text{-}Glu(OR^1)\text{-}Asn\text{-}OR^2 \qquad II$$

worin Y A,

A-Ala-,

A-Glu($OR^1$)-Ala-,

A-Glu($OR^1$)-Glu($OR^1$)-Ala-,

A-Val-Glu($OR^1$)-Glu($OR^1$)-Ala- oder

A-Val-Val-Glu($OR^1$)-Glu($OR^1$)-Ala;

A Wasserstoff oder eine herkömmliche α-Aminoschutzgruppe und

$R^1$ und $R^2$ herkömmliche Carboxyschutzgruppen bedeuten,

in an sich bekannter Weise die Schutzgruppen abspaltet, und erwünschtenfalls den erhaltenen Stoff in ein physiologisch verträgliches Salz überführt.

11. Pharmazeutische Präparate enthaltend eine Verbindung gemäss Anspruch 1 und einen geeigneten Träger.

12. Verwendung von Verbindungen gemäss Anspruch 1 als pharmazeutische Wirkstoffe.

***

## Patentansprüche für Oesterreich

1. Verfahren zur Herstellung von Peptiden der Formel

X-Glu-Asn-OH I

worin X H,
H-Ala-,
H-Glu-Ala-,
H-Glu-Glu-Ala-,
H-Val-Glu-Glu-Ala- oder
H-Val-Val-Glu-Glu-Ala- bedeutet,

und ihrer physiologisch verträglichen Salze, dadurch gekennzeichnet, dass man aus einer Verbindung der Formel

$$Y\text{-}Glu(OR^1)\text{-}Asn\text{-}OR^2 \qquad II$$

worin Y A,
A-Ala-,
$A\text{-}Glu(OR^1)\text{-}Ala\text{-}$,
$A\text{-}Glu(OR^1)\text{-}Glu(OR^1)\text{-}Ala\text{-}$,
$A\text{-}Val\text{-}Glu(OR^1)\text{-}Glu(OR^1)\text{-}Ala\text{-}$ oder
$A\text{-}Val\text{-}Val\text{-}Glu(OR^1)\text{-}Glu(OR^1)\text{-}Ala$;
A Wasserstoff oder eine herkömmliche α-Aminoschutzgruppe und
$R^1$ und $R^2$ herkömmliche Carboxyschutzgruppen bedeuten,

in an sich bekannter Weise die Schutzgruppen abspaltet, und erwünschtenfalls den erhaltenen Stoff in ein physiologisch verträgliches Salz überführt.

2. Verfahren gemäss Anspruch 1, dadurch gekennzeichnet, dass A t-Butoxycarbonyl und $R^1$ und $R^2$ je Benzyl oder t-Butyl bedeuten.

3. Verfahren gemäss Anspruch 1, dadurch gekennzeichnet, dass A Benzyloxycarbonyl und $R^1$ und $R^2$ je Benzyl oder t-Butyl bedeuten.

4. Verfahren gemäss einem der Ansprüche 1 bis 3, dadurch gekennzeichnet, dass die Abspaltung der Schutzgruppen durch Behandeln mit wasserfreiem Fluorwasserstoff in Gegenwart von Anisol, durch katalytische Hydrierung und/oder durch Behandeln mit Trifluoressigsäure in Methylenchlorid erfolgt.

***